# EUROPEAN PATENT APPLICATION

(11) **EP 1 264 823 A1**
(43) Date of publication of application: **11.12.2002**
(21) Application number: 01113967.2
(22) Date of filing: 08.06.2001
(51) Int. Cl.: C07C 253/00, C07C 255/19, C07D 231/38

(54) **Process for the preparation of 2,3-dicyanopropionates**

(71) Applicant: Novartis AG, 4056 Basel (CH)
(72) Inventor: Schüle, Gunter, 4056 Basel (CH)
(74) Representative: Becker, Konrad

(57) **Abstract**

The invention relates to a process for the preparation of a compound of formula 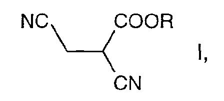 wherein R represents branched or unbranched (C₁-C₆-alkoxy)₁₋₁₀-C₂-C₆-alkyl, C₃-C₈-cycloalkyl, allyl, propargyl, phenyl or phenyl-C₁-C₆-alkyl; or a salt thereof; which comprises the reaction of a cyanoacetate of formula 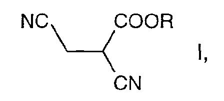 wherein R is defined as in formula I, with formaldehyde or a source thereof in the presence of a cyanide salt. The compounds of formula I are useful as intermediates in the synthesis of pesticidally active compounds.

## Description

This invention relates to a process for preparing certain 2,3-dicyanopropionic esters and their use in the synthesis of pesticides and pesticide intermediates.

Ethyl 2,3-dicyanopropionate was first synthesized by Higson and Thorpe in 1906 (J. Chem. Soc. 89, 1460 (1906)) and later by Dickinson (J. Am. Chem. Soc. 82, 6132 (1960)) by reaction of formaldehyde cyanohydrin with the sodium salt of ethyl cyanoacetate. Due to the inherent problem of having to isolate the highly watersoluble and fairly unstable intermediate formaldehyde cyanohydrin, Hawkins et al. (WO 97/32843, 12.9.97) suggested a process avoiding the cyanohydrin altogether by reacting an alkyl cyanoacetate in the presence of sodium cyanide with formaldehyde. This procedure gives the corresponding 2,3-dicyanopropionic alkyesters in good yields; however, it requires absolutely waterfree conditions. Thus, none of these attempts is totally satisfactory.

The present invention, in a first aspect, provides a process for the preparation of 2,3-dicyanopropionic esters which avoids the use of formaldehyde cyanohydrin, simplifies waterfree conditions and gives the required product in high yield and with high purity.

Therefore, the present invention provides a process for the preparation of a compound of formula 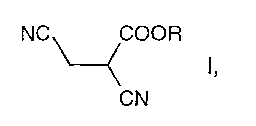 wherein R represents branched or unbranched (C₁-C₆-alkoxy)₁₋₁₀-C₂-C₆-alkyl, C₃-C₈-cycloalkyl, allyl, propargyl, phenyl or phenyl-C₁-C₆-alkyl; or a salt thereof;
which comprises the reaction of a cyanoacetate of formula 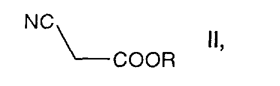 wherein R is defined as in formula I, with formaldehyde or a source thereof in the presence of a cyanide salt.

Preferably, R represents unbranched (C₁-C₂-alkoxy)₁₋₂-C₂-C₄-alkyl, C₄-C₆-cycloalkyl or phenyl-C₁-C₂-alkyl, more preferably unbranched C₁-C₂-alkoxy-C₂-C₄-alkyl or phenyl-C₁-alkyl, most preferably unbranched C₁-alkoxy-C₂-C₄-alkyl.

Suitable salts of cyanides encompass metal salts and organic salts such as tetraalkylammonium, preferably tetrabutylammonium, cyanides. Suitable metal salts are alkali or alkaline earth metal cyanide salts, particularly alkali metal cyanides, especially potassium or sodium cyanide.

The product may be isolated as the alkali or alkaline earth metal salt. Preferably, the reaction mixture is acidified, for example with a mineral acid such as sulfuric of hydrochloric acid, to yield the compound of fomula I in the free form.

The reaction may be carried out with formaldehyde itself; however, it is usually preferred to use the polymerised form known as the commercially available paraformaldehyde.

The reaction partners can be reacted with one another as they are, i.e. without the addition of a solvent or diluent, e.g. in the melt. In most cases, however, the addition of an inert solvent or diluent, or a mixture thereof, is of advantage. Examples of such solvents or diluents are: aromatic, aliphatic and alicyclic hydrocarbons and halogenated hydrocarbons, such as benzene, toluene, xylene, mesitylene, tetraline, chlorobenzene, dichlorobenzene, bromobenzene, petroleum ether, hexane, cyclohexane, dichloromethane, trichloromethane, tetrachloromethane, dichloroethane, trichloroethene or tetrachloroethene; alcohols, such as methanol, ethanol, propanol, isopropanol, butanol, ethylene glycol, polyethylene glycol, ethylene glycol monomethyl ether, or glycerol; ethers, such as diethyl ether, dipropyl ether, diisopropyl ether, dibutyl ether, tert-butyl methyl ether, ethylene glycol monomethyl ether, ethylene glycol monoethyl ether, ethylene glycol dimethylether, dimethoxydiethylether, tetrahydrofuran or dioxane; ketones such as acetone, methyl ethyl ketone or methyl isobutyl ketone; amides such as N,N-dimethylformamide, N,N-diethylformamide, N,N-dimethylacetamide, N-methylpyrrolidone or hexamethylphosphoric acid triamide; nitriles such as acetonitrile or propionitrile; and sulphoxides, such as dimethyl sulphoxide. Preferably, the reaction is carried out in an alcohol or ether, more preferably in an alcohol. Especially preferred solvents is ethylene glycol monomethyl ether.

In a preferred process, a molar equivalent of a compound of formula II is mixed with about a molar equivalent of a cyanide salt and the mixture gradually treated with about a molar equivalent of formaldehyde in its paraformaldehyde precursor form in ethylene glycol monomethyl ether under unhydrous conditions at temperatures of about 0 to about 160°C or at the reflux temperature of the solvent, particularly at temperatures of about 0 to 40°C.

In a second aspect, the present invention provides a process for the generation of the compound of formula 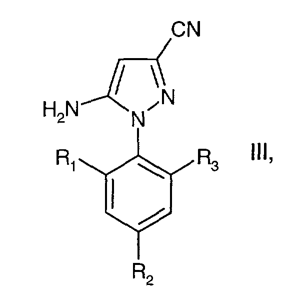 wherein R₁, R₂ and R₃ represent independently of each other halogen, C₁-C₄-haloalkyl, C₁-C₄-haloalkoxy or SF₅; preferably R₁ and R₃ halogen and R₂ trifluoromethyl, trifluoromethoxy or SF₅; which process comprises reacting the compound of formula I with the diazonium salt of a compound of formula 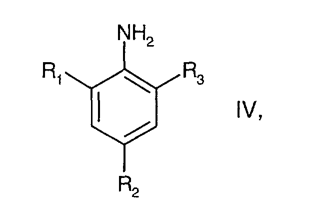 wherein R₁, R₂ and R₃ are defined as in formula III, to give a compound of formula 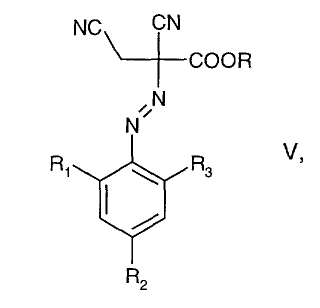 wherein R₁, R₂ and R₃ are defined as in formula III; and cyclisation of the compound of formula V by means of a base, preferably ammonia.

In this process, the reaction partners can be reacted with one another as they are, i.e. without the addition of a solvent or diluent, e.g. in the melt. In most cases, however, the addition of an inert solvent or diluent, or a mixture thereof, is of advantage. Examples of such solvents or diluents are given above. Preferably, the complete reaction sequence is carried out in an alcohol or ether, more preferably in an alcohol. Especially preferred solvents is ethylene glycol monomethyl ether.

In a preferred process, the diazonium salt of a compound of formula IV may be prepared using common diazotising agents known in the literature, preferably with a molar equivalent of sodium nitrite and a mineral acid such as hydrochloric or sulfuric acid at a temperature of about -10 to about 40°C, particularly from about 0 to about 10°C. Preferably, the diazonium salt is generated in situ in order to ensure its quick reaction with the compound of formula I before being reduced by the alcoholic solution. Subsequent hydrolysis of the ester group and cyclisation is preferably carried out with an aqueous base, particularly aqueous ammonia.

In a third aspect, the present invention provides a process for the in situ generation of the compound of formula I and the subsequent preparation of the compound of formula III, wherein R₁, R₂ and R₃ represent independently of each other halogen, C₁-C₄-haloalkyl, C₁-C₄-haloalkoxy or SF₅; preferably R₁ and R₃ halogen and R₂ trifluoromethyl, trifluoromethoxy or SF₅; which process comprises
a) reacting a cyanoacetate of formula II with a cyanide salt and formaldehyde or a source thereof, to give a compound of formula I;
b) reacting the compound of formula I thus obtained with the diazonium salt of a compound of formula IV, wherein R₁, R₂ and R₃ are defined as in formula III, to give a compound of formula V, wherein R₁, R₂ and R₃ are defined as in formula III; and
c) cyclisation of the compound of formula V by means of a base, preferably ammonia.

In this process, the reaction partners can be reacted with one another as they are, i.e. without the addition of a solvent or diluent, e.g. in the melt. In most cases, however, the addition of an inert solvent or diluent, or a mixture thereof, is of advantage. Examples of such solvents or diluents are given above. Preferably, the complete reaction sequence is carried out in an alcohol or ether, more preferably in an alcohol. Especially preferred solvents is ethylene glycol monomethyl ether.

In a preferred process, reaction step a) is carried out as described above, whereby the product of step a) is generally acidified with an alcoholic solution of a mineral acid. The diazonium salt of a compound of formula IV required in step b) may be prepared using common diazotising agents known in the literature, preferably with a molar equivalent of sodium nitrite and a mineral acid such as hydrochloric or sulfuric acid at a temperature of about -10 to about 40°C, particularly from about 0 to about 10°C. Preferably, the diazonium salt is generated in situ in order to ensure its quick reaction with the compound of formula I before being reduced by the alcoholic solution. Subsequent hydrolysis of the ester group and cyclisation in step c) is preferably carried out with an aqueous base, particularly aqueous ammonia.

The compound of formula III is an important intermediate for the preparation of pesticidally active compounds.

The following non-limiting examples illustrate the present invention.

### Example 1: 2-Methoxyethyl 2,3-dicyanopropionate

2-Methoxyethylcyanoacetate (3.5 ml) and sodium cyanide (1.72 g) are suspended in 2-methoxyethanol (18 ml) and treated with paraformaldehyde (1.05 g) in portions under ice-cooling. After addition the mixture is left to warm up to room temperature and stirred for 6 hours. The reddish mixture is then acidified to pH 2 with ca. 4 ml aqueous hydrochloric acid under ice-cooling and finally the solvent evaporated at 55°C in vacuo. The residue is extracted with diethyl ether/water, the water phase is washed with diethyl ether, the combined organic phases are washed twice with water and then with a saturated sodium chloride solution, dried, filtered and evaporated to dryness in vacuo. The residue is distilled at 180°C and 0.08 mbar to give the title compound as brownish oil.

### Example 2: 5-amino-3-cyano-1-(2,6-dichloro-4-trifluoromethylphenyl)-pyrazole

To 2-Methoxyethylcyanoacetate (491 mg), dissolved in 2-methoxyethanol (4 ml), aqueous hydrochloric acid (0.65 ml) and then 2,6-dichloro-4-trifluoromethylaniline (575 mg), dissolved in 2-methoxyethanol (2 ml), is added under a nitrogen atmosphere. At 5 to 10°C, an aqueous solution (1 ml) of sodium nitrite (242 mg) is added dropwise within 2 minutes and the mixture stirred for 3 hours under ice-cooling. The mixture is then set to pH 9 by addition of aqueous ammonia (25%) and stirred overnight at ambient temperature. After evaporation of the solvent the residue is extracted with ethyl acetate / water, and the organic phase washed twice with water and with a saturated sodium chloride solution, dried, filtered and evaporated to dryness in vacuo. The residue is recrystallized from toluene to give the title compound, m.p. 141-2°C.

### Example 3: 5-amino-3-cyano-1-(2,6-dichloro-4-trifluoromethylphenyl)-pyrazole

2-Methoxyethylcyanoacetate (2.7 ml) and sodium cyanide (1.33 g) are suspended in 2-methoxyethanol (14 ml) and treated with paraformaldehyde (0.81 g) in portions under ice-cooling. After addition the mixture is left to warm up to room temperature and stirred for 6 hours. The reddish mixture is then acidified to pH 2 with ca. 3 ml aqueous hydrochloric acid under ice-cooling and 2,6-dichloro-4-trifluoromethylaniline (5.75 g), dissolved in 2-methoxyethanol (20 ml), is added within 5 minutes. At 5 to 10°C, an aqueous solution (10 ml) of sodium nitrite (2.42 g) is added dropwise within 2 minutes and the mixture stirred for 3 hours under ice-cooling. The mixture is then set to pH 9 by addition of aqueous ammonia (25%) and stirred overnight at ambient temperature. After evaporation of the solvent the residue is extracted with ethyl acetate / water, and the organic phase washed twice with water and with a saturated sodium chloride solution, dried, filtered and evaporated to dryness in vacuo. The residue is recrystallized from toluene to give the title compound, m.p. 141-2°C.

## Claims

1. A process for the preparation of a compound of formula 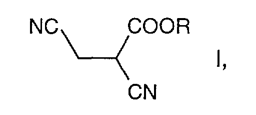 wherein R represents branched or unbranched (C₁-C₆-alkoxy)₁₋₁₀-C₂-C₆-alkyl, C₃-C₈-cycloalkyl, allyl, propargyl, phenyl or phenyl-C₁-C₆-alkyl; or a salt thereof;
which comprises the reaction of a cyanoacetate of formula 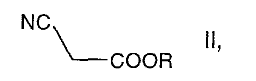 wherein R is defined as in formula I, with formaldehyde or a source thereof in the presence of a cyanide salt.

2. A process according to claim 1 wherein the reaction is carried out at temperatures of about 0 to about 160°C or at the reflux temperature of the solvent.

3. A process for the preparation of a compound of formula 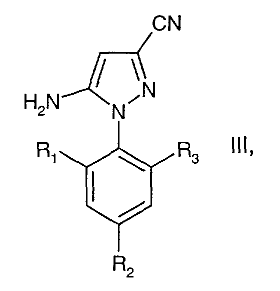 wherein R₁, R₂ and R₃ represent independently of each other halogen, C₁-C₄-haloalkyl, C₁-C₄-haloalkoxy or SF₅; which process comprises reacting the compound of formula I with the diazonium salt of a compound of formula 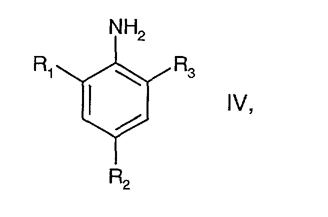 wherein R₁, R₂ and R₃ are defined as in formula III, to give a compound of formula 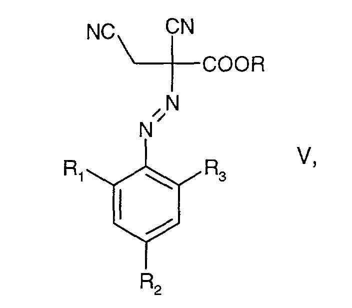 wherein R₁, R₂ and R₃ are defined as in formula III; and cyclisation of the compound of formula V by means of a base.

4. A process for the preparation of a compound of formula III, wherein R₁, R₂ and R₃ represent independently of each other halogen, C₁-C₄-haloalkyl, C₁-C₄-haloalkoxy or SF₅; which process comprises
a) reacting a cyanoacetate of formula II with a cyanide salt and formaldehyde or a source thereof, to give a compound of formula I;
b) reacting the compound of formula I thus obtained with the diazonium salt of a compound of formula IV, wherein R₁, R₂ and R₃ are defined as in formula III, to give a compound of formula V, wherein R₁, R₂ and R₃ are defined as in formula III; and
c) cyclisation of the compound of formula V by means of a base.

5. A process according to any one of the previous claims, wherein R represents unbranched C₁-C₂-alkoxy-C₂-C₄-alkyl or phenyl-C₁-alkyl.

6. A process according to any one of the previous claims, wherein the source of formaldehyde is paraformaldehyde.

7. A process according to any one of the previous claims, wherein the reaction is performed in presence of ethylene glycol monomethyl ether as a solvent.
